# EUROPEAN PATENT SPECIFICATION

(11) **EP 2 215 040 B1**
(45) Date of publication and mention of the grant of the patent: **01.06.2016**
(21) Application number: 08840130.2
(22) Date of filing: 18.10.2008
(51) Int. Cl.: C07C 67/08, C07C 69/732, C07C 67/31, C07C 269/06, C07C 41/48, C07C 43/315, C07C 271/12

(54) **PROCESS FOR PREPARING (S) 2-(2-CHLOROPHENYL)-2-HYDROXY-ETHYL CARBAMATE**
VERFAHREN ZUR HERSTELLUNG VON (S) 2-(2-CHLOROPHENYL)-2-HYDROXY-ETHYL CARBAMAT
PROCÉDÉ DE PRÉPARATION DU CARBAMATE DE (S) 2-(2-CHLOROPHÉNYL)-2-HYDROXY-ÉTHYLE

(30) Priority: 18.10.2007 US 980877 P
(43) Date of publication of application: 11.08.2010
(73) Proprietor: SK Biopharmaceuticals Co., LTD, Jongro-gu Seoul 110-110 (KR)
(72) Inventor: PORSTMANN, Frank, 8234 Stetten (CH); MEIER, Thomas, 8200 Schaffhausen (CH)
(74) Representative: Warner, James Alexander
(86) International application number: PCT/IB2008/003300
(87) International publication number: WO 2009/050587

(56) References cited:
- DE-A1- 3 145 436
- US-A- 4 156 791
- US-A- 4 237 138
- US-A- 5 250 744
- US-A- 6 127 412

## Description

### Brief Description of the Invention

The present invention relates to a new process for preparing a (S)-(+)- 2-(substituted phenyl) -2 hydroxy-ethyl carbamate as defined herein.

### Background of the Invention

2- (substituted phenyl) -2 hydroxy-ethyl carbamates and their isomers have been described in US 5,698,588, US 5,854,283, US 6,127,412, US 6,103,759, WO 97/26241, and WO 03/053916 as compounds useful for treating disorders of the central nervous system. Said disorders include convulsions, epilepsy, stroke, muscles spasms, neuropathic pain, and migraine. A preferred disorder is epilepsy.

### Summary of the Invention

Disclosed is a process of preparing a compound of Formula I: 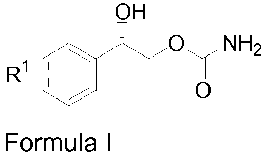 wherein:
R¹ is selected from the group consisting of Cl, F, and Br;
said process comprising the use of lithium borohydride to reduce the ester intermediate of Formula II, to an alcohol of Formula III, which is carried on in subsequent reactions to become a compound of Formula I; 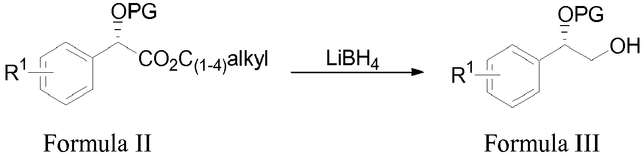 wherein PG is a protecting group derived from an ether, wherein said ether is selected from the group consisting of 2-methoxy-1-propene, dihydropyran, methyl vinyl ether, ethyl vinyl ether, and 5,6-dihydro-4-methoxy-2H-pyran, wherein said compound and said process are further defined herein.

The present invention relates to a process of preparing (S)-carbamic acid 2-(2-chloro-phenyl)-2-hydroxy-ethyl ester: 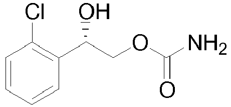 said process comprising the following steps:
a) esterification of (S)-(2-chloro-phenyl)-hydroxy-acetic acid, 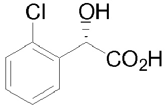 (S)-(2-chloro-phenyl)-hydroxy-acetic acid
   with a C(₁-₄)alcohol, selected from the group consisting of: methanol, ethanol, n-propanol, iso-propanol, n-butanol, iso-butanol, sec-butanol, and tert-butanol: wherein said esterification is characterized by the use of: 0.025 to 0.4 equivalents phosphorous oxychloride to obtain an ester of Formula IV. 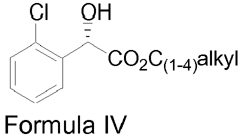
b) protection of the alcohol moiety of a compound of Formula IV, wherein said protection is characterized by the use of acidic catalysis controlled by left over acid from phosphorous oxychloride from the previous reaction and an ether selected from the group consisting of: 2-methoxy-1-propene, dihydropyran, methyl vinyl ether, ethyl vinyl ether, and 5,6-dihydro-4-methoxy-2H-pyran to obtain a compound of Formula II, wherein PG is a protecting group derived from an ether, wherein said ether is selected from the group consisting of 2-methoxy-1-propene, dihydropyran, methyl vinyl ether, ethyl vinyl ether, and 5,6-dihydro-4-methoxy-2H-pyran. 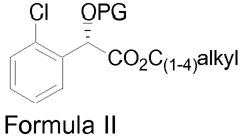
c) reduction of the ester intermediate of Formula II, to an alcohol of Formula III, wherein said reduction is characterized by the use of lithium borohydride 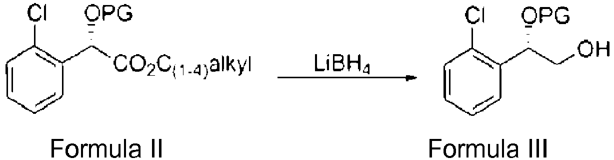 wherein PG is as defined in Formula II.

### Detailed Description of the Invention

Disclosed is a process of preparing a compound of Formula I: 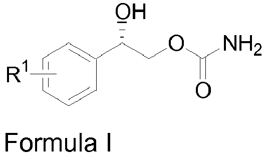 wherein:
R¹ is selected from the group consisting of Cl, F, and Br;
said process comprising the use of lithium borohydride to reduce the ester intermediate of Formula II, to an alcohol of Formula III, which is carried on in subsequent reactions to become a compound of Formula I. 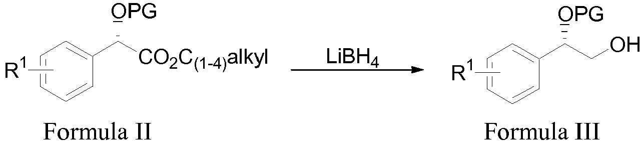 wherein PG is a protecting group derived from an ether, wherein said ether is selected from the group consisting of 2-methoxy-1-propene, dihydropyran, methyl vinyl ether, ethyl vinyl ether, and 5,6-dihydro-4-methoxy-2H-pyran, wherein said compound and said process are further defined herein.

The present invention relates to a process of preparing (S)-carbamic acid 2-(2-chloro-phenyl)-2-hydroxy-ethyl ester: 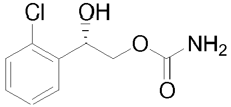
(S)-carbamic acid 2-(2-chloro-phenyl)-2-hydroxy-ethyl ester
said process comprising the use of lithium borohydride to reduce the ester intermediate of Formula II, to an alcohol of Formula III, which is carried on in subsequent reactions to become (S)-carbamic acid 2-(2-chloro-phenyl)-2-hydroxy-ethyl ester. 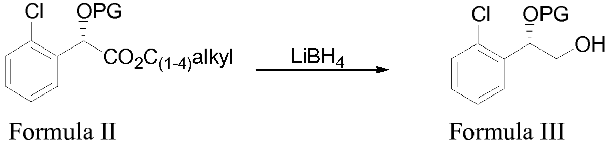 wherein PG is as defined in Formula II.

The process of preparing (S)-carbamic acid 2-(2-chloro-phenyl)-2-hydroxy-ethyl ester: 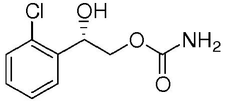 (S)-carbamic acid 2-(2-chloro-phenyl)-2-hydroxy-ethyl ester of the present invention comprises the use of the steps a, b, and c as described below:
a) esterification of (S)-(2-chloro-phenyl)-hydroxy-acetic acid, 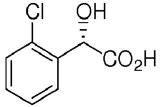 (S)-(2-chloro-phenyl)-hydroxy-acetic acid
   with a C(₁-₄)alcohol, selected from the group consisting of: methanol, ethanol, n-propanol, iso-propanol, n-butanol, iso-butanol, sec-butanol, and tert-butanol: wherein said esterification is characterized by the use of: 0.025 to 0.4 equivalents phosphorous oxychloride to obtain an ester of Formula IV. 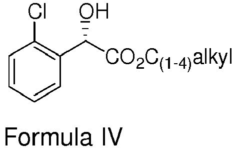
b) protection of the alcohol moiety of a compound of Formula IV, wherein said protection is characterized by the use of acidic catalysis controlled by left over acid from phosphorous oxychlolide from the previous reaction and an ether selected from the group consisting of: 2-methoxy-1-propene, dihydropyran, methyl vinyl ether, ethyl vinyl ether, and 5,6-dihydro-4-methoxy-2H-pyran to obtain a compound of Formula II, 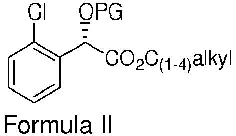 wherein PG is as defined in Formula II.
c) Use of lithium borohydride to reduce the ester intermediate of Formula II, to an alcohol of Formula III, which is carried on in subsequent reactions to become (S)-carbamic acid 2-(2-chloro-phenyl)-2-hydroxy-ethyl ester. 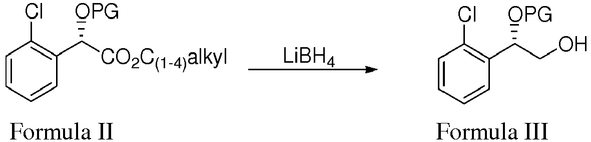 wherein PG is as defined in Formula II.

In one embodiment, the present invention relates to a process of preparing (S)-carbamic acid 2-(2-chloro-phenyl)-2-hydroxy-ethyl ester 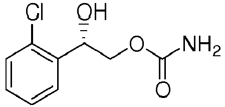 (S)-carbamic acid 2-(2-chloro-phenyl)-2-hydroxy-ethyl ester said process comprising the use of the steps a, b, c, d, e, f, and g as described below:
a) esterification of (S)-(2-chloro-phenyl)-hydroxy-acetic acid, 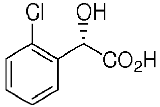 (S)-(2-chloro-phenyl)-hydroxy-acetic acid
   with a C(₁-₄)alcohol, selected from the group consisting of: methanol, ethanol, n-propanol, iso-propanol, n-butanol, iso-butanol, sec-butanol, and tert-butanol: wherein said esterification is characterized by the use of: 0.025 to 0.4 equivalents phosphorous oxychloride to obtain an ester of Formula IV. 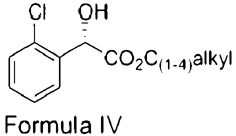
b) protection of the alcohol moiety of a compound of Formula IV, wherein said protection is characterized by the use of acidic catalysis controlled by left over acid from phosphorous oxychlolide from the previous reaction and an ether selected from the group consisting of: 2-methoxy-1-propene, dihydropyran, methyl vinyl ether, ethyl vinyl ether, and 5,6-dihydro-4-methoxy-2H-pyran to obtain a compound of Formula II 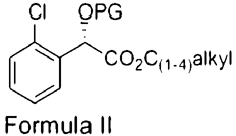 wherein PG is as defined in Formula II.
c) Use of lithium borohydride to reduce the ester intermediate of Formula II, to an alcohol of Formula III. 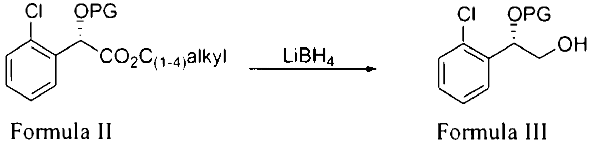 wherein PG is as defined in Formula II.
d) Reaction of an alcohol of Formula III 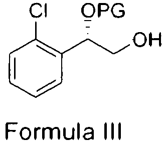 with a carbonyl compound, 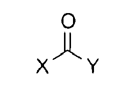 wherin X and Y are leaving groups independently selected from the group consisting of bromo, chloro, imidazolyl, and phenoxy;
   wherein said reaction is characterized by a reaction temperature: -10 to 65 °C, to yield a carbonyl compound of Formula V. 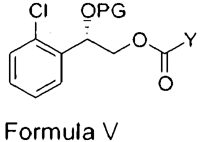
e) Reaction of a compound of Formula V with ammonium hydroxide or ammonia, wherein said reaction is characterized by reaction temperature: 0 to 55 °C to form the carbamate of Formula VI. 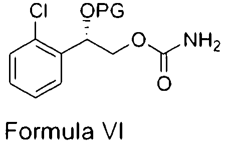
f) Deprotection of the carbamate of Formula VI, wherein said deprotection is characterized by the use of an acid selected from the group consisting of phosphoric acid, hydrochloric acid, and sulfuric acid to form (S)-carbamic acid 2-(2-chloro-phenyl)-2-hydroxy-ethyl ester 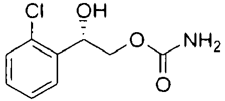 (S)-carbamic acid 2-(2-chloro-phenyl)-2-hydroxy-ethyl ester
g) Recrystalization of (S)-carbamic acid 2-(2-chloro-phenyl)-2-hydroxy-ethyl ester, wherein said recrystalization is characterized by a controlled cooling gradient.

In another embodiment, the present invention relates to a process of preparing (S)-carbamic acid 2-(2-chloro-phenyl)-2-hydroxy-ethyl ester 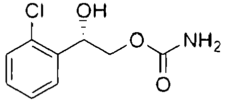 (S)-carbamic acid 2-(2-chloro-phenyl)-2-hydroxy-ethyl ester said process comprising the use of the steps a, b, and c as described below:
a) Methyl esterification of (S)-(2-chloro-phenyl)-hydroxy-acetic acid, 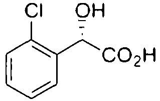 (S)-(2-chloro-phenyl)-hydroxy-acetic acid
   wherein said esterification is characterized by the use of 0.1 equivalents phosphorous oxychloride to obtain (S)-(2-chloro-phenyl)-hydroxy-acetic acid methyl ester. 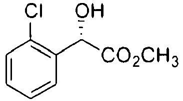 (S)-(2-chloro-phenyl)-hydroxy-acetic acid methyl ester
b) protection of (S)-(2-chloro-phenyl)-hydroxy-acetic acid methyl ester, wherein said protection is characterized by the use of acidic catalysis controlled by left over acid from phosphorous oxychlolide from the previous reaction and 2-methoxy-1-propene, to obtain (S)-(2-chloro-phenyl)-(1-methoxy-1-methyl-ethoxy)-acetic acid methyl ester. 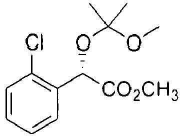 (S)-(2-chloro-phenyl)-(1-methoxy-1-methyl-ethoxy)-acetic acid methyl ester
c) reduction of (S)-(2-chloro-phenyl)-(1-methoxy-1-methyl-ethoxy)-acetic acid methyl ester, wherein said reduction is characterized by the use of LiBH₄, to obtain (S)-2-(2-chloro-phenyl)-2-(1-methoxy-1-methyl-ethoxy)-ethanol, which is carried on in subsequent reactions to become (S)-carbamic acid 2-(2-chloro-phenyl)-2-hydroxy-ethyl ester. 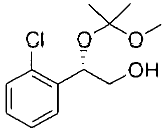 (S)-2-(2-chloro-phenyl)-2-(1-methoxy-1-methyl-ethoxy)-ethanol

In another embodiment of the invention the present invention relates to a process of preparing (S)-carbamic acid 2-(2-chloro-phenyl)-2-hydroxy-ethyl ester: 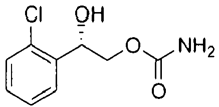 (S)-carbamic acid 2-(2-chloro-phenyl)-2-hydroxy-ethyl ester
said process comprising the use of the steps a, b, c, e, f, and g as described below:
a) methyl esterification of (S)-(2-chloro-phenyl)-hydroxy-acetic acid: 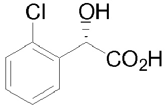 (S)-(2-chloro-phenyl)-hydroxy-acetic acid
   wherein said methyl esterification is characterized by the use of:
   10 equivalents of methanol,
   0.1 equivalents of phosphorous oxychloride,
   reaction temperature: 40 to 50 °C,
   partial concentration, and
   toluene co-solvent;
   to obtain (S)-(2-chloro-phenyl)-hydroxy-acetic acid methyl ester. 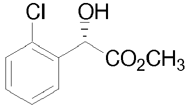 (S)-(2-chloro-phenyl)-hydroxy-acetic acid methyl ester
b) protection of (S)-(2-chloro-phenyl)-hydroxy-acetic acid methyl ester, wherein said protection is characterized by the use of:
   acidic catalysis controlled by left over acid from phosphorous oxychloride from the previous step,
   addition of 1.6 equivalents isopropenyl methyl ether to reaction mixture, and
   1 extraction with water;
   to obtain (S)-(2-chloro-phenyl)-(1-methoxy-1-methyl-ethoxy)-acetic acid methyl ester. 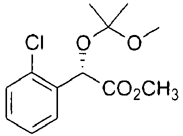 (S)-(2-chloro-phenyl)-(1-methoxy-1-methyl-ethoxy)-acetic acid methyl ester
c) reduction of (S)-(2-chloro-phenyl)-(1-methoxy-1-methyl-ethoxy)-acetic acid methyl ester, wherein said reduction is characterized by the use of:
   LiBH₄,
   solvents: Aprotic solvents like ethers e.g. THF, diethylether and the like,
   hydrocarbons like e.g. toluene,
   workup with ketones like e.g. acetone,
   reaction temperature: 40 to 50 °C,
   distillation of THF and acetone, and and isopropanol (the last one formed during workup procedure by reduction of acetone)
   2 extractions with sodium hydroxide solution and NaCl-solution; to obtain (S)-2-(2-chloro-phenyl)-2-(1-methoxy-1-methyl-ethoxy)-ethanol. 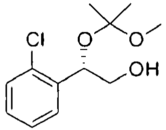 (S)-2-(2-chloro-phenyl)-2-(1-methoxy-1-methyl-ethoxy)-ethanol
e) reaction of (S)-imidazole-1-carboxylic acid 2-(2-chloro-phenyl)-2-(1-methoxy-1-methyl-ethoxy)-ethyl ester 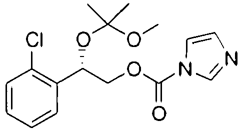 (S)-imidazole-1-carboxylic acid 2-(2-chloro-phenyl)-2-(1-methoxy-1-methyl-ethoxy)-ethyl ester
   with ammonium hydroxide, wherein said reaction is characterized by
   1.60 equivalents of ammonium hydroxide, and
   reaction temperature: 40 to 50 °C
   to form (S)-carbamic acid 2-(2-chloro-phenyl)-2-(1-methoxy-1-methyl-ethoxy)-ethyl ester. 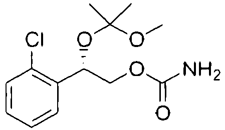 (S)-carbamic acid 2-(2-chloro-phenyl)-2-(1-methoxy-1-methyl-ethoxy)-ethyl ester
f) deprotection of (S)-carbamic acid 2-(2-chloro-phenyl)-2-(1-methoxy-1-methyl-ethoxy)-ethyl ester, wherein said deprotection is characterized by
   addition of 2 equivalents of aq. sulfuric acid, and
   reaction temperature: 40 to 50 °C
   to form (S)-carbamic acid 2-(2-chloro-phenyl)-2-hydroxy-ethyl ester. 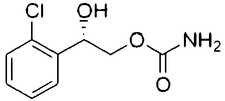 (S)-carbamic acid 2-(2-chloro-phenyl)-2-hydroxy-ethyl ester
g) recrystallization of (S)-carbamic acid 2-(2-chloro-phenyl)-2-hydroxy-ethyl ester, wherein said recrystalization is characterized by
   methanol / water 1 : 4, and
   use of a controlled temperature gradient.

### Examples:

The following examples illustrate embodiments of the invention as they apply to the synthesis of 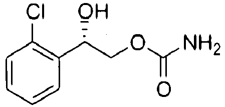 can we use the (S)-carbamic acid 2-(2-chloro-phenyl)-2-(1-methoxy-1-methyl-ethoxy)-ethyl ester instead of the formula in the text. . Use of any one of the following procedures, or an equivalent thereof yields unexpected advantages that are described below.

### Example 1

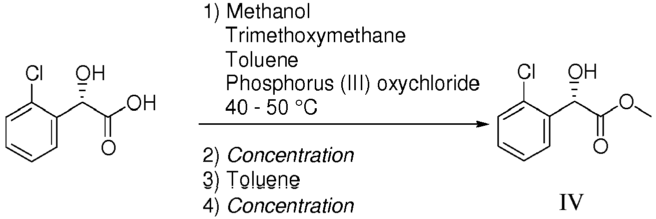

A reaction vessel was charged with 300 kg (1607.8 mol) of (S)-2-chloromandelic acid, 519 kg (16199 mol) of methanol, 24.6 kg (160.4 mol) of phosphoric trichloride, 190 kg (1790.4 mol) of trimethoxymethane and 672 kg of toluene, and heated to 40 to 50 °C, whereupon a clear solution was formed. The solution was partially concentrated in vacuum until approximately 708 kg of solvents were removed. An additional 708 kg of toluene were added and the distillation in vacuum was repeated until 708 kg of solvents were again removed. Ester IV was transferred to the following reaction step as solution in toluene.

Partial concentration of the toluene solution, as opposed to full removal of the methanol as reagent / solvent and toluene as solvent, provided the benefit of avoiding the reverse reaction, and thereby increasing the reaction yield. In addition, it was discovered that less than equimolar amounts like 0.025 -0.4 equivalents of POCl₃ could be used as acid, rather than 2.8 equivalents of HCl. The use of a substochiometrical amounts of POCl₃ has advantages which include: increased safety by avoiding the need to destroy a large amount of acid after distillation, reduction of cycle time, decreased use of reagents, and an overall simplification of the process (no gaseous HCl addition).

### Example 2

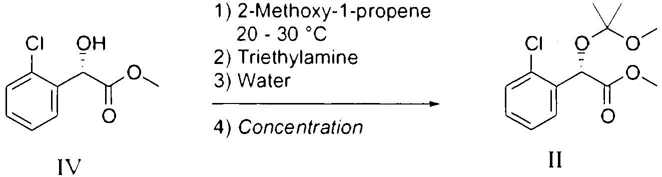

The reaction mixture was cooled to 5 to 15 °C. A mass of 184 kg (2552 mol) of 2-methoxy-1-propene was slowly added at 5 to 30 °C. The resulting solution was stirred for 30 to 120 min at 20 to 30 °C before 24.6 kg (243.1 mol) of anhydrous triethylamine were added. After the pH had been checked to be in the range of 8 to 11, 270 kg of water were added, whereupon a biphasic mixture was formed. The emulsion was stirred for 10 to 30 min, before the layers were separated. The lower aqueous layer was discarded (approximately 305 kg). The organic layer was partially concentrated in vacuum until approximately 300 kg of solvents were removed. Ester II was transferred to the following reaction step as solution.

The discovery that pH control for acidic catalysis controlled by left over acid from phosphorous oxychloride from the previous step could be used in place of new POCl₃ resulted in several simplifications of the process, including the following: avoidance of the need to add additional phosphorous oxychloride, avoidance of the need to monitor and control the pH for optimum ketal formation, and eliminating the need for reactor change which resulted in reduction of cycle time. In addition, it was discovered that this reaction did not require multiple washings and drying over Na₂SO₄; rather, a single water wash with azeotropic drying was found to be sufficient.

### Example 3

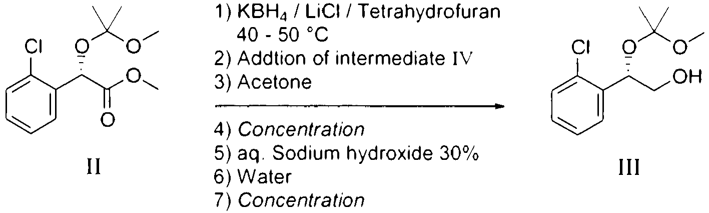

A dry vessel was charged with 91.8 kg (1702.2 mol) potassium borohydrate, 72.0 kg (1698.9 mol) of lithium chloride, and 516 kg of tetrahydrofuran. The resulting suspension was stirred at 40 to 50 °C for 1 to 2 h before the solution of ester II was added over 90 to 180 min at 40 to 50 °C. After the addition, the reaction mixture was stirred at 40 to 50 °C for an additional 2.5 to 3.5 h. At the same temperature 271 kg of acetone were added slowly over 60 to 120 min, followed by 240 kg of toluene, and the mixture was stirred for an additional 30 to 60 min at 40 to 50 °C. Vacuum distillation was then used to remove 786 kg of solvents. Sodium hydroxide solution (approximately 30% (w/w), 278 kg, 2085 mol) was added, followed by 557 kg of purified water. The resulting emulsion was stirred for 20 to 40 min at 40 to 50 °C, and the layers were separated. The lower aqueous layer was discarded (approximately 997 kg). The organic layer was diluted with 240 kg toluene, treated with 300 kg purified water, and 30 kg of sodium chloride. The emulsion is stirred at 40 to 50 °C for 10 to 30 min and the layers were again separated. The aqueous layer (approximately 360 kg) is discarded. The organic layer was partially concentrated in vacuum until approximately 600 kg of solvents were removed. Alcohol III was transferred to the following reaction step as solution.

Use of LiBH₄, either as solid reagent or as solution in an appropriate solvent or formed in situ from potassium borohydride and lithium chloride, as the reducing agent resulted in several improvements to the procedure, including: eliminating the need to use the solvent tetraethyleneglycol dimethyl ether -which can be difficult to remove, lower reaction temperature, increased volume yield, and elimination of the need for Na₂SO₄ drying and filtration. In addition use of LiBH₄ resulted in excellent and unexpected selectivity of reduction. Avoided undesirable side reactions include removal of the protecting group and/or dehalogenation.

### Comparative Example 4

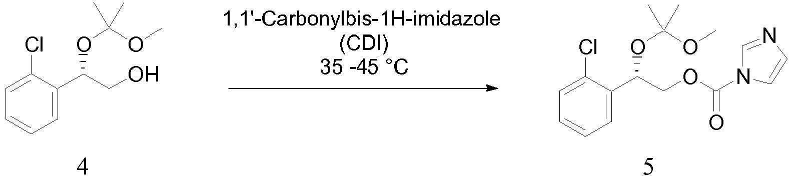

A reaction vessel was charged with 326 kg (2010.5) mol 1,1'-carbonylbis-1H-imidazole and

540 kg toluene. The suspension was vigorously stirred for 10 to 30 min before the solution of alcohol III was added slowly over 30 min at 15 to 45 °C, forming a nearly clear solution. Imidazolide V was transferred to the following reaction step as solution in toluene.

Using a temperature range of 15 to 45 °C resulted in consistently complete conversion and increased volume yield, when compared to the same reaction run at a temperature range of 0 to 20 °C.

### Example 5

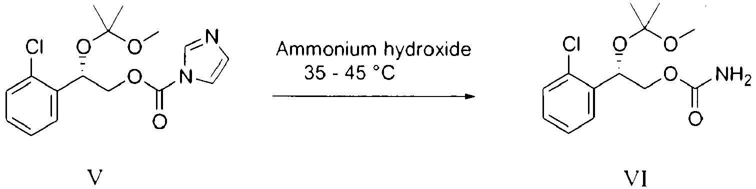

The solution from the previous step was stirred at 35 to 45 °C for 30 to 60 min before 175 kg of ammonium hydroxide solution (25% (w/v), 1.6 eq.) was added slowly within 90 to 180 min at 35 to 45 °C. The biphasic reaction mixture was stirred at 35 to 45 °C for an additional 45 to 75 min. Carbamate VI was transferred to the following step in the biphasic reaction mixture.

Use of 1.6 (1.44-1.77) equivalents of ammonium hydroxide rather than 10 equivalents allowed the reaction mixture to be transferred directly to the following step with no aqueous extractions. The new procedure had the benefits of complete conversion with a low incidence of side reactions, no product losses in the aqueous layers, and increased volume yield.

### Example 6

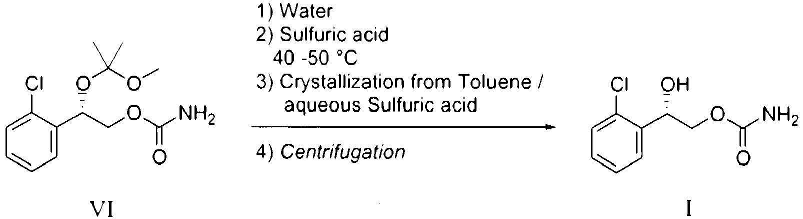

The biphasic reaction mixture containing the carbamate VI was treated with 392 kg of purified water, followed by the addition of 311 kg (3171.2 mol, 2 equivalents) of sulfuric acid at 15 to 50 °C, followed by an additional 43.5 kg of purified water. The emulsion was stirred at 40 to 50 °C for 45 to 75 min whereupon the crude carbamate I precipitated. The suspension was cooled to 10 to 20 °C, followed by centrifugation. The wet product (approximately 360 kg) was washed with 360 kg of purified water in several portions and dried in a tray dryer (drying cabinet) or a mechanical dryer in vacuum and 65 to 75 °C for 0.5 to 24 h.

Use of 2 equivalents of sulfuric acid allowed the combination of carbamate formation in example 5 with the procedure in example 6. Further the use of 2 equivalents of sulfuric acid at 40 to 50 °C rather than 0.73 equivalents of HCl at 20 to 30 °C resulted in increased yield from 83.7% to 91.6%. Volume yield increased from 70 g/L to 146 g/L, and cycle time was reduced.

### Example 7

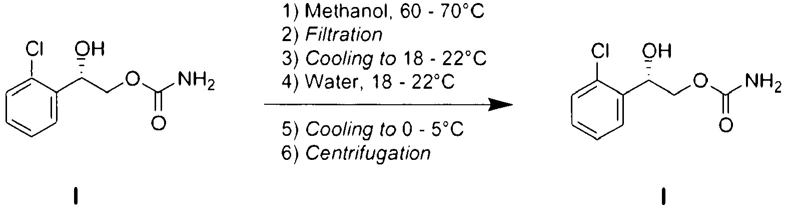

Carbamate I was dissolved in methanol at reflux temperature (∼65 °C) and filtered via a 0.45 µm filter cartridge into a preheated reactor. The solution was cooled with a defined cooling gradient to 18 to 22 °C during which the product started to crystallize.

Water was added to the suspension, until the methanol/water ratio is 1:4. The suspension was further cooled with a defined gradient to 0 - 5 °C. The suspension was filtered and the resulting cake was washed with water. The wet product was dried under vacuum at 48 - 52 °C in a drying cabinet or in a mechanical dryer.

Use of a controlled cooling gradient resulted in a slightly increased volume yield from 118 g/L to 123 g/L and control of the polymorphs.

## Claims

1. A process of preparing (S)-carbamic acid 2-(2-chloro-phenyl)-2-hydroxy-ethyl ester: 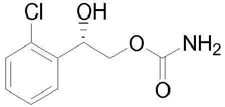 said process comprising the following steps:
a) esterification of (S)-(2-chloro-phenyl)-hydroxy-acetic acid, 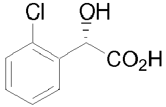 (S)-(2-chloro-phenyl)-hydroxy-acetic acid
with a C(₁-₄)alcohol, selected from the group consisting of: methanol, ethanol, n-propanol, iso-propanol, n-butanol, iso-butanol, sec-butanol, and tert-butanol: wherein said esterification is **characterized by** the use of: 0.025 to 0.4 equivalents phosphorous oxychloride to obtain an ester of Formula IV. 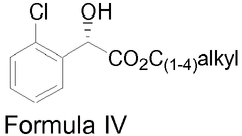
b) protection of the alcohol moiety of a compound of Formula IV, wherein said protection is **characterized by** the use of acidic catalysis controlled by left over acid from phosphorous oxychloride from the previous reaction and an ether selected from the group consisting of: 2-methoxy-1-propene, dihydropyran, methyl vinyl ether, ethyl vinyl ether, and 5,6-dihydro-4-methoxy-2H-pyran to obtain a compound of Formula II, wherein PG is a protecting group derived from an ether, wherein said ether is selected from the group consisting of 2-methoxy-1-propene, dihydropyran, methyl vinyl ether, ethyl vinyl ether, and 5,6-dihydro-4-methoxy-2H-pyran. 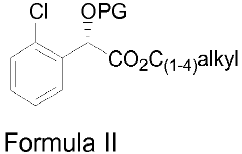
c) reduction of the ester intermediate of Formula II, to an alcohol of Formula III, wherein said reduction is **characterized by** the use of lithium borohydride 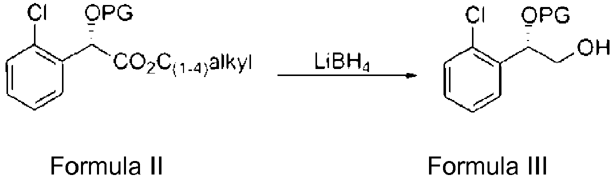 wherein PG is as defined in Formula II.

2. The process of Claim 1 further comprising steps d, e, f, and g as shown below:
d) reaction of an alcohol of Formula III 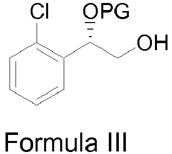 with a carbonyl compound, 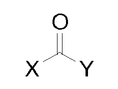 wherein X and Y are leaving groups independently selected from the group consisting of bromo, chloro, imidazolyl, and phenoxy;
wherein said reaction is **characterized by** a reaction temperature: -10 to 65 °C, to yield a carbonyl compound of Formula V. 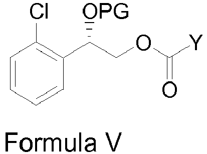
e) reaction of a compound of Formula V with ammonium hydroxide or ammonia, wherein said reaction is **characterized by** reaction temperature: 0 to 55 °C to form the carbamate of Formula VI. 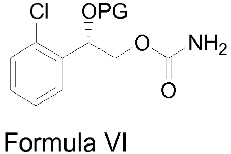
f) deprotection of the carbamate of Formula VI, wherein said deprotection is **characterized by** the use of an acid selected from the group consisting of phosphoric acid, hydrochloric acid, and sulfuric acid to form (S)-carbamic acid 2-(2-chloro-phenyl)-2-hydroxy-ethyl ester 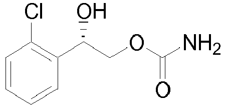 (S)-carbamic acid 2-(2-chloro-phenyl)-2-hydroxy-ethyl ester
g) recrystalization of (S)-carbamic acid 2-(2-chloro-phenyl)-2-hydroxy-ethyl ester, wherein said recrystalization is **characterized by** a controlled cooling gradient.

3. The process of Claim 1 comprising preparing (S)-carbamic acid 2-(2-chloro-phenyl)-2-hydroxy-ethyl ester 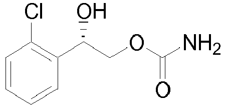 (S)-carbamic acid 2-(2-chloro-phenyl)-2-hydroxy-ethyl ester said process comprising the use of the steps a, b, and c as described below:
a) methyl esterification of (S)-(2-chloro-phenyl)-hydroxy-acetic acid, 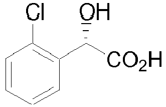 (S)-(2-chloro-phenyl)-hydroxy-acetic acid
wherein said esterification is **characterized by** the use of 0.1 equivalents phosphorous oxychloride to obtain (S)-(2-chloro-phenyl)-hydroxy-acetic acid methyl ester. 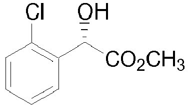 (S)-(2-chloro-phenyl)-hydroxy-acetic acid methyl ester
b) protection of (S)-(2-chloro-phenyl)-hydroxy-acetic acid methyl ester, wherein said protection is **characterized by** the use of acidic catalysis controlled by left over acid from phosphorous oxychloride from the previous reaction and 2-methoxy-1-propene, to obtain (S)-(2-chloro-phenyl)-(1-methoxy-1-methyl-ethoxy)-acetic acid methyl ester. 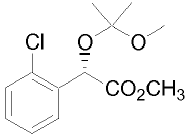 (S)-(2-chloro-phenyl)-(1-methoxy-1-methyl-ethoxy)-acetic acid methyl ester
c) reduction of (S)-(2-chloro-phenyl)-(1-methoxy-1-methyl-ethoxy)-acetic acid methyl ester, wherein said reduction is **characterized by** the use of LiBH₄, to obtain (S)-2-(2-chloro-phenyl)-2-(1-methoxy-1-methyl-ethoxy)-ethanol 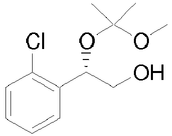 (S)-2-(2-chloro-phenyl)-2-(1-methoxy-1-methyl-ethoxy)-ethanol.

4. The process of Claim 1 comprising preparing (S)-carbamic acid 2-(2-chloro-phenyl)-2-hydroxy-ethyl ester: 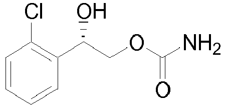 (S)-carbamic acid 2-(2-chloro-phenyl)-2-hydroxy-ethyl ester said process comprising the use of the steps a, b, c, e, f, and g as described below:
a) methyl esterification of (S)-(2-chloro-phenyl)-hydroxy-acetic acid: 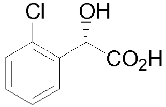 (S)-(2-chloro-phenyl)-hydroxy-acetic acid
wherein said methyl esterification is **characterized by** the use of:
10 equivalents of methanol,
0.1 equivalents of phosphorous oxychloride,
reaction temperature: 40 to 50 °C,
partial concentration, and
toluene co-solvent;
to obtain (S)-(2-chloro-phenyl)-hydroxy-acetic acid methyl ester. 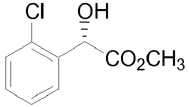 (S)-(2-chloro-phenyl)-hydroxy-acetic acid methyl ester
b) protection of (S)-(2-chloro-phenyl)-hydroxy-acetic acid methyl ester, wherein said protection is **characterized by** the use of:
acidic catalysis controlled by left over acid from phosphorous oxychloride from the previous step,
addition of 1.6 equivalents isopropenyl methyl ether to reaction mixture, and
1 extraction with water;
to obtain (S)-(2-chloro-phenyl)-(1-methoxy-1-methyl-ethoxy)-acetic acid methyl ester. 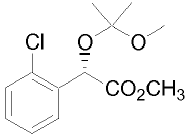 (S)-(2-chloro-phenyl)-(1-methoxy-1-methyl-ethoxy)-acetic acid methyl ester
c) reduction of (S)-(2-chloro-phenyl)-(1-methoxy-1-methyl-ethoxy)-acetic acid methyl ester, wherein said reduction is **characterized by** the use of:
LiBH₄,
THF as solvent
workup with acetone reaction temperature: 40 to 50 °C,
distillation of THF and acetone and Isopropanol which is formed during workup procedure by reduction of acetone and
2 extractions with sodium hydroxide solution and NaCl-solution;
to obtain (S)-2-(2-chloro-phenyl)-2-(1-methoxy-1-methyl-ethoxy)-ethanol. 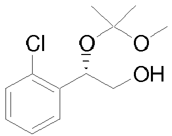
(S)-2-(2-chloro-phenyl)-2-(1-methoxy-1-methyl-ethoxy)-ethanol
e) reaction of (S)-imidazole-1-carboxylic acid 2-(2-chloro-phenyl)-2-(1-methoxy-1-methyl-ethoxy)-ethyl ester with ammonium hydroxide, wherein said reaction is **characterized by**
1.60 equivalents of ammonium hydroxide, and
reaction temperature: 40 to 50 °C
to form (S)-carbamic acid 2-(2-chloro-phenyl)-2-(1-methoxy-1-methyl-ethoxy)-ethyl ester. 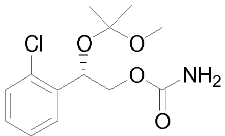 (S)-carbamic acid 2-(2-chloro-phenyl)-2-(1-methoxy-1-methyl-ethoxy)-ethyl ester
f) deprotection of (S)-carbamic acid 2-(2-chloro-phenyl)-2-(1-methoxy-1-methyl-ethoxy)-ethyl ester, wherein said deprotection is **characterized by**
addition of 2 equivalents of aq. sulfuric acid, and
reaction temperature: 40 to 50 °C
to form (S)-carbamic acid 2-(2-chloro-phenyl)-2-hydroxy-ethyl ester. 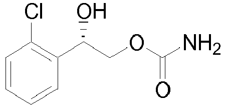 (S)-carbamic acid 2-(2-chloro-phenyl)-2-hydroxy-ethyl ester
g) recrystallization of (S)-carbamic acid 2-(2-chloro-phenyl)-2-hydroxy-ethyl ester, wherein said recrystalization is **characterized by**
methanol / water 1 : 4, and
use of a controlled temperature gradient.

## Patentansprüche

1. Verfahren zur Herstellung von (S)-Carbamidsäure-2-(2-chlorphenyl)-2-hydroxyethylester: 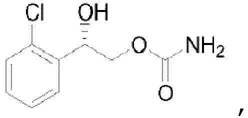 wobei das Verfahren die folgenden Schritte umfasst:
a) Veresterung von (S)-(2-Chlorphenyl)hydroxyessigsäure, 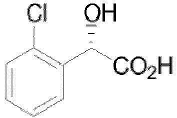 (S)-(2-Chlorphenyl)hydroxyessigsäure mit einem C₍₁₋₄₎Alkohol, ausgewählt aus der Gruppe bestehend aus: Methanol, Ethanol, n-Propanol, Isopropanol, n-Butanol, Isobutanol, sek.-Butanol und tert.-Butanol: wobei die Veresterung durch die Verwendung von 0,025 bis 0,4 Äquivalenten Phosphoroxychlorid unter Erhalt eines Esters der Formel IV gekennzeichnet ist. 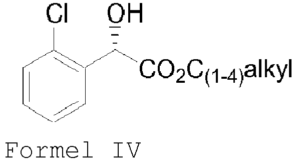
b) Schützen der Alkoholgruppierung einer Verbindung der Formel IV, wobei das Schützen durch die Verwendung von durch übriggebliebene Säure von Phosphoroxychlorid aus der vorhergehenden Reaktion gesteuerter Säurekatalyse und einem aus der aus 2-Methoxy-1-propen, Dihydropyran, Methylvinylether, Ethylvinylether und 5,6-Dihydro-4-methoxy-2H-pyran bestehenden Gruppe ausgewählten Ether unter Erhalt einer Verbindung der Formel II gekennzeichnet ist, wobei PG für eine von einem Ether abgeleitete Schutzgruppe steht, wobei der Ether aus der aus 2-Methoxy-1-propen, Dihydropyran, Methylvinylether, Ethylvinylether und 5,6-Dihydro-4-methoxy-2H-pyran bestehenden Gruppe ausgewählt ist. 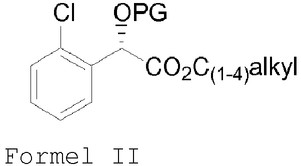
c) Reduktion der Ester-Zwischenstufe der Formel II zu einem Alkohol der Formel III, wobei die Reduktion durch die Verwendung von Lithiumborhydrid gekennzeichnet ist, 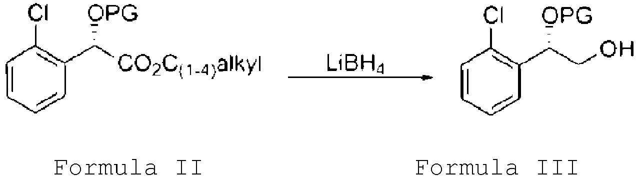 wobei PG die bei Formel II angegebene Bedeutung hat.

2. Verfahren nach Anspruch 1, ferner umfassend die Schritte d, e, f und g wie nachfolgend dargestellt:
d) Umsetzung eines Alkohols der Formel III 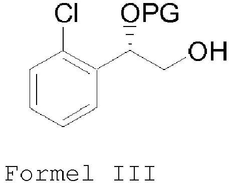 mit einer Carbonylverbindung, 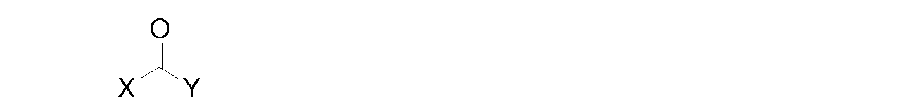 wobei X und Y für Abgangsgruppen stehen, die unabhängig aus der aus Brom, Chlor, Imidazolyl und Phenoxy bestehenden Gruppe ausgewählt sind;
wobei die Umsetzung durch eine Reaktionstemperatur -10 bis 65° C gekennzeichnet ist, unter Erhalt einer Carbonylverbindung der Formel V. 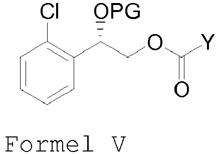
e) Umsetzung einer Verbindung der Formel V mit Ammoniumhydroxid oder Ammoniak, wobei die Umsetzung durch Reaktionstemperatur 0 bis 55° C gekennzeichnet ist, unter Bildung des Carbamats der Formel VI. 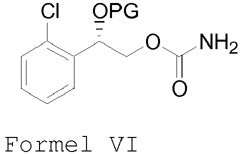
f) Entschützen des Carbamats der Formel VI, wobei das Entschützen durch die Verwendung einer aus der aus Phosphorsäure, Salzsäure und Schwefelsäure bestehenden Gruppe ausgewählten Säure unter Bildung von (S)-Carbamidsäure-2-(2-chlorphenyl)-2-hydroxyethylester gekennzeichnet ist 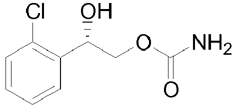 (S)-Carbamidsäure-2-(2-chlorphenyl)-2-hydroxy-ethylester
g) Umkristallisierung von (S)-Carbamidsäure-2-(2-chlorphenyl)-2-hydroxyethylester, wobei die Umkristallisierung durch einen gesteuerten Abkühlungsgradienten gekennzeichnet ist.

3. Verfahren nach Anspruch 1, umfassend Herstellen von (S)-Carbamidsäure-2-(2-chlorphenyl)-2-hydroxy-ethylester, 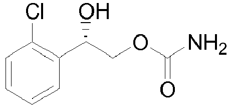 (S)-Carbamidsäure-2-(2-chlorphenyl)-2-hydroxy-ethylester
wobei das Verfahren die Verwendung der Schritte a, b und c wie nachfolgend beschrieben umfasst:
a) Methylveresterung von (S)-(2-Chlorphenyl)-hydroxyessigsäure, 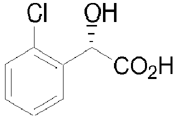 (S)-(2-Chlorphenyl)hydroxyessigsäure wobei die Veresterung durch die Verwendung von 0,1 Äquivalenten Phosphoroxychlorid unter Erhalt von (S)-(2-Chlorphenyl)hydroxyessigsäuremethylester gekennzeichnet ist. 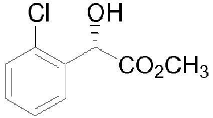 (S)-(2-Chlorphenyl)hydroxyessigsäuremethylester
b) Schützen von (S)-(2-Chlorphenyl)hydroxyessigsäuremethylester, wobei das Schützen durch die Verwendung von durch übriggebliebene Säure von Phosphoroxychlorid aus der vorhergehenden Reaktion gesteuerter Säurekatalyse und 2-Methoxy-1-propen gekennzeichnet ist, unter Erhalt von (S)-(2-Chlorphenyl)-(1-methoxy-1-methylethoxy)essigsäuremethylester. 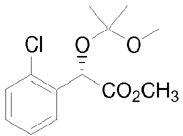 (S)-(2-Chlorphenyl)-(1-methoxy-1-methylethoxy)-essigsäuremethylester
c) Reduktion von (S)-(2-Chlorphenyl)-(1-methoxy-1-methylethoxy)essigsäuremethylester, wobei die Reduktion durch die Verwendung von LiBH₄ gekennzeichnet ist, unter Erhalt von (S)-2-(2-Chlorphenyl)-2-(1-methoxy-1-methylethoxy)ethanol 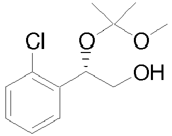 (S)-2-(2-Chlorphenyl)-2-(1-methoxy-1-methyl-ethoxy)ethanol.

4. Verfahren nach Anspruch 1, umfassend Herstellen von (S)-Carbamidsäure-2-(2-chlorphenyl)-2-hydroxy-ethylester, 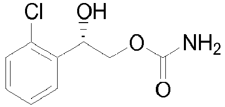 (S)-Carbamidsäure-2-(2-chlorphenyl)-2-hydroxy-ethylester
wobei das Verfahren die Verwendung der Schritte a, b, c, e, f und g wie nachfolgend beschrieben umfasst:
a) Methylveresterung von (S)-(2-Chlorphenyl)-hydroxyessigsäure, 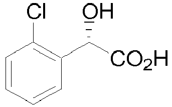 (S)-(2-Chlorphenyl)hydroxyessigsäure
wobei die Methylveresterung durch die Verwendung von:
10 Äquivalenten Methanol,
0,1 Äquivalenten Phosphoroxychlorid,
Reaktionstemperatur: 40 bis 50 °C,
Teilkonzentration und
Toluol-Cosolvens
gekennzeichnet ist;
unter Erhalt von (S)-(2-Chlorphenyl)hydroxyessigsäuremethylester. 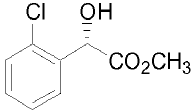 (S)-(2-Chlorphenyl)hydroxyessigsäuremethylester
b) Schützen von (S)-(2-Chlorphenyl)hydroxyessigsäuremethylester, wobei das Schützen durch die Verwendung von:
durch übriggebliebene Säure von Phosphoroxychlorid aus dem vorhergehenden Schritt gesteuerter Säurekatalyse,
Zugabe von 1,6 Äquivalenten Isopropenylmethylether zum Reaktionsansatz und
1 Extraktion mit Wasser
gekennzeichnet ist;
unter Erhalt von (S)-(2-Chlorphenyl)-(1-methoxy-1-methylethoxy)essigsäuremethylester. 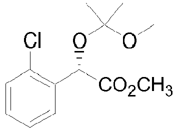 (S)-(2-Chlorphenyl)-(1-methoxy-1-methylethoxy)-essigsäuremethylester
c) Reduktion von (S)-(2-Chlorphenyl)-(1-methoxy-1-methylethoxy)essigsäuremethylester, wobei die Reduktion durch die Verwendung von:
LiBH₄,
THF als Lösungsmittel Aufarbeiten mit Aceton Reaktionstemperatur: 40 bis 50 °C,
Destillieren von THF und Aceton und Isopropanol, das während der Aufarbeitung durch Reduktion von Aceton gebildet wird, und
2 Extraktionen mit Natriumhydroxidlösung und NaCl-Lösung
gekennzeichnet ist;
unter Erhalt von (S)-2-(2-Chlorphenyl)-2-(1-methoxy-1-methylethoxy)ethanol. 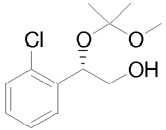 (S)-2-(2-Chlorphenyl)-2-(1-methoxy-1-methyl-ethoxy)ethanol
e) Umsetzung von (S)-Imidazol-1-carbonsäure-2-(2-chlorphenyl)-2-(1-methoxy-1-methylethoxy)ethylester mit Ammoniumhydroxid, wobei die Umsetzung durch
1,60 Äquivalente Ammoniumhydroxid und
Reaktionstemperatur: 40 bis 50 °C
gekennzeichnet ist,
unter Bildung von (S)-Carbamidsäure-2-(2-chlorphenyl)-2-(1-methoxy-1-methylethoxy)ethylester. 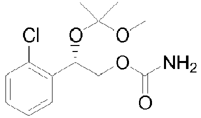 (S)-Carbamidsäure-2-(2-chlorphenyl)-2-(1-methoxy-1-methylethoxy)ethylester
f) Entschützen von (S)-Carbamidsäure-2-(2-chlorphenyl)-2-(1-methoxy-1-methylethoxy)ethylester, wobei das Entschützen durch
Zugabe von 2 Äquivalenten wässrige Schwefelsäure und
Reaktionstemperatur: 40 bis 50 °C
gekennzeichnet ist,
unter Bildung von (S)-Carbamidsäure-2-(2-chlorphenyl)-2-hydroxyethylester. 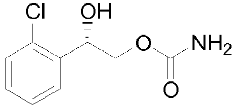 (S)-Carbamidsäure-2-(2-chlorphenyl)-2-hydroxyethylester
g) Umkristallisierung von (S)-Carbamidsäure-2-(2-chlorphenyl)-2-hydroxyethylester, wobei die Umkristallisierung durch
Methanol/Wasser 1:4 und
Verwendung eines gesteuerten Temperaturgradienten
gekennzeichnet ist.

## Revendications

1. Procédé de préparation d'ester (S)-2-(2-chlorophényl)-2-hydroxy-éthylique d'acide carbamique : 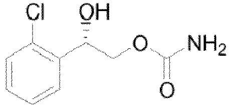 ledit procédé comprenant les étapes suivantes :
a) estérification d'acide (S)-(2-chloro-phényl)-hydroxy-acétique, 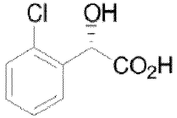 acide (S)-(2-chloro-phényl)-hydroxy-acétique
avec un alcool en C₍₁₋₄₎, choisi dans le groupe constitué de : méthanol, éthanol, n-propanol, iso-propanol, n-butanol, iso-butanol, sec-butanol, et tert-butanol : dans lequel ladite estérification est **caractérisée par** l'utilisation de : 0,025 à 0,4 équivalent d'oxychlorure phosphoreux pour obtenir un ester de formule IV, 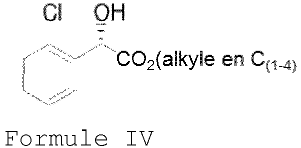
b) protection du fragment alcool d'un composé de formule IV, ladite protection étant **caractérisée par** l'utilisation d'une catalyse acide contrôlée par l'acide résiduel de l'oxychlorure phosphoreux de la réaction précédente et un éther choisi dans le groupe constitué de : 2-méthoxy-1-propène, dihydropyrane, éther méthyl-vinylique, éther éthyl-vinylique, et 5,6-dihydro-4-méthoxy-2H-pyrane pour obtenir un composé de formule II, PG étant un groupe protecteur dérivé d'un éther, ledit éther étant choisi dans le groupe constitué des 2-méthoxy-1-propène, dihydropyrane, éther méthyl-vinylique, éther éthyl-vinylique, et 5,6-dihydro-4-méthoxy-2H-pyrane, 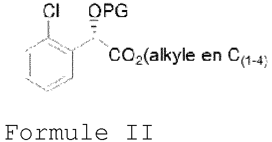
c) réduction de l'ester intermédiaire de formule II, en alcool de formule III, ladite réduction étant **caractérisée par** l'utilisation de borohydrure de lithium 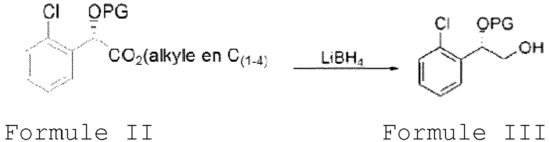 PG étant tel que défini dans la formule II.

2. Procédé de la revendication 1 comprenant en outre les étapes d, e, f, et g telles que décrites ci-dessous :
d) réaction d'un alcool de formule III 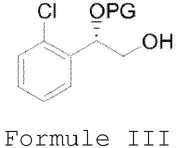 avec un composé carbonyle, 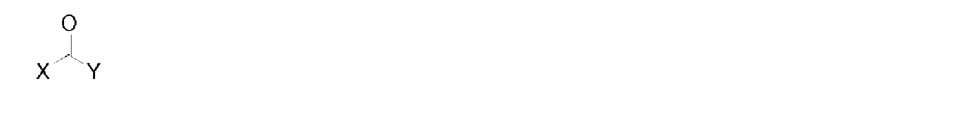 dans lequel X et Y sont des groupes partants indépendamment choisis dans le groupe constitué de bromo, chloro, imidazolyle, et phénoxy ;
ladite réaction étant **caractérisée par** une température de réaction de : -10 à 65 °C, pour obtenir un composé carbonyle de formule V, 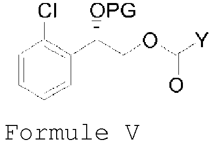
e) réaction d'un composé de formule V avec de l'hydroxyde d'ammonium ou de l'ammoniac, ladite réaction étant **caractérisée par** une température de réaction de : 0 à 55 °C pour former le carbamate de formule VI, 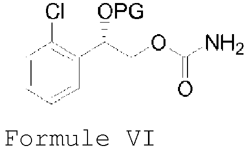
f) déprotection du carbamate de formule VI, ladite déprotection étant **caractérisée par** l'utilisation d'un acide choisi dans le groupe constitué de l'acide phosphorique, l'acide chlorhydrique, et l'acide sulfurique pour former l'ester (S)-2-(2-chloro-phényl)-2-hydroxy-éthylique d'acide carbamique 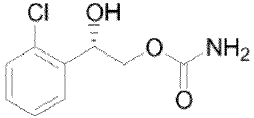 ester (S)-2-(2-chloro-phényl)-2-hydroxy-éthylique d'acide carbamique
g) recristallisation de l'ester (S)-2-(2-chloro-phényl)-2-hydroxy-éthylique d'acide carbamique, ladite recristallisation étant **caractérisée par** un gradient de refroidissement contrôlé.

3. Procédé de la revendication 1 comprenant la préparation d'ester (S)-2-(2-chloro-phényl)-2-hydroxy-éthylique d'acide carbamique 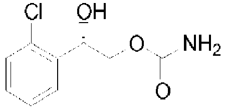 ester (S)-2-(2-chloro-phényl)-2-hydroxy-éthylique d'acide carbamique
ledit procédé comprenant l'utilisation des étapes a, b, et c telles que décrites ci-dessous :
a) estérification méthylique d'acide (S)-(2-chloro-phényl)-hydroxy-acétique, 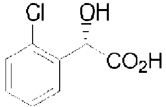 acide (S)-(2-chloro-phényl)-hydroxy-acétique
ladite estérification étant **caractérisée par** l'utilisation de 0,1 équivalent d'oxychlorure phosphoreux pour obtenir l'ester méthylique d'acide (S)-(2-chloro-phényl)-hydroxy-acétique, 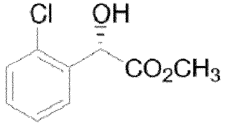 ester méthylique d'acide (S)-(2-chloro-phényl)-hydroxy-acétique
b) protection d'ester méthylique d'acide (S)-(2-chloro-phényl)-hydroxy-acétique, ladite protection étant **caractérisé par** l'utilisation d'une catalyse acide contrôlée par l'acide résiduel de l'oxychlorure phosphoreux de la réaction précédente et de 2-méthoxy-1-propène, pour obtenir l'ester méthylique d'acide (S)-(2-chloro-phényl)-(1-méthoxy-1-méthyl-éthoxy)-acétique, 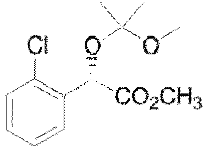 ester méthylique d'acide (S)-(2-chloro-phényl)-(1-méthoxy-1-méthyl-éthoxy)-acétique
c) réduction d'ester méthylique d'acide (S)-(2-chloro-phényl)-(1-méthoxy-1-méthyl-éthoxy)-acétique, ladite réduction étant **caractérisée par** l'utilisation de LiBH₄, pour obtenir le (S)-2-(2-chloro-phényl)-2-(1-méthoxy-1-méthyl-éthoxy)-éthanol 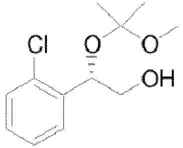 (S)-2-(2-chloro-phényl)-2-(1-méthoxy-1-méthyl-éthoxy)-éthanol.

4. Procédé de la revendication 1 comprenant la préparation d'ester (S)-2-(2-chloro-phényl)-2-hydroxy-éthylique d'acide carbamique : 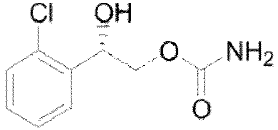 ester (S)-2-(2-chloro-phényl)-2-hydroxy-éthylique d'acide carbamique
ledit procédé comprenant l'utilisation des étapes a, b, c, e, f, et g telles que décrites ci-dessous :
a) estérification méthylique d'acide (S)-(2-chloro-phényl)-hydroxy-acétique : 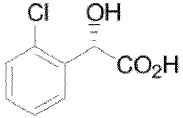 acide (S)-(2-chloro-phényl)-hydroxy-acétique
ladite estérification méthylique étant **caractérisée par** l'utilisation de :
10 équivalents de méthanol,
0,1 équivalent d'oxychlorure phosphoreux,
température de réaction : 40 à 50 °C,
concentration partielle, et
co-solvant : toluène ;
pour obtenir l'ester méthylique d'acide (S)-(2-chloro-phényl)-hydroxy-acétique, 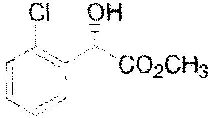 ester méthylique d'acide (S)-(2-chloro-phényl)-hydroxy-acétique
b) protection d'ester méthylique d'acide (S)-(2-chloro-phényl)-hydroxy-acétique, ladite protection étant **caractérisée par** l'utilisation de :
catalyse acide contrôlée par l'acide résiduel de l'oxychlorure phosphoreux de l'étape précédente,
ajout de 1,6 équivalent d'éther isopropényl-méthylique au mélange de réaction, et
1 extraction avec de l'eau ;
pour obtenir l'ester méthylique d'acide (S)-(2-chloro-phényl)-(1-méthoxy-1-méthyl-éthoxy)-acétique, 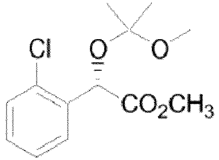 ester méthylique d'acide (S)-(2-chloro-phényl)-(1-méthoxy-1-méthyl-éthoxy)-acétique
c) réduction d'ester méthylique d'acide (S)-(2-chloro-phényl)-(1-méthoxy-1-méthyl-éthoxy)-acétique, ladite réduction étant **caractérisée par** l'utilisation de :
LiBH₄,
THF en tant que solvant
lavage avec de l'acétone
température de réaction : 40 à 50 °C,
distillation de THF et de l'acétone et de l'isopropanol qui est formé pendant la procédure de lavage par réduction de l'acétone et
2 extractions avec une solution d'hydroxyde de sodium et une solution de NaCl ;
pour obtenir le (S)-2-(2-chloro-phényl)-2-(1-méthoxy-1-méthyl-éthoxy)-éthanol, 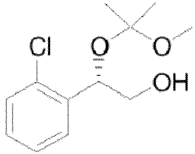 (S)-2-(2-chloro-phényl)-2-(1-méthoxy-1-méthyl-éthoxy)-éthanol
e) réaction d'ester (S)-2-(2-chloro-phényl)-2-(1-méthoxy-1-méthyl-éthoxy)-éthylique d'acide imidazole-1-carboxylique avec de l'hydroxyde d'ammonium, ladite réaction étant **caractérisée par**
1,60 équivalent d'hydroxyde d'ammonium, et
température de réaction: 40 à 50 °C
pour former l'ester (S)-2-(2-chloro-phényl)-2-(1-méthoxy-1-méthyl-éthoxy)-éthylique d'acide carbamique, 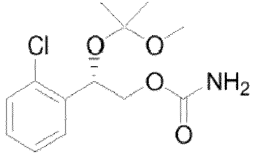 Ester (S)-2-(2-chloro-phényl)-2-(1-méthoxy-1-méthyl-éthoxy)-éthyl d'acide carbamique
f) déprotection de l'ester (S)-2-(2-chloro-phényl)-2-(1-méthoxy-1-méthyl-éthoxy)-éthylique d'acide carbamique, ladite déprotection étant **caractérisée par** l'ajout de 2 équivalents d'acide sulfurique aq., et température de réaction: 40 à 50 °C
pour former l'ester (S)-2-(2-chloro-phényl)-2-hydroxy-éthylique d'acide carbamique 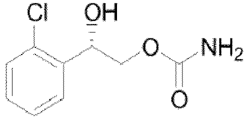 ester (S)-2-(2-chloro-phényl)-2-hydroxy-éthylique d'acide carbamique
g) recristallisation de l'ester (S)-2-(2-chloro-phényl)-2-hydroxy-éthylique d'acide carbamique, ladite recristallisation étant **caractérisée par** méthanol / eau 1 : 4, et l'utilisation d'un gradient de température contrôlé.
